# EUROPEAN PATENT SPECIFICATION

(11) **EP 3 760 184 B1**
(45) Date of publication and mention of the grant of the patent: **15.02.2023**
(21) Application number: 19184324.2
(22) Date of filing: 04.07.2019
(51) Int. Cl.: A61K 8/24, A61K 8/34, A61K 8/36, A61K 8/44, A61K 8/64, A61K 8/67, A61K 8/73, A61Q 19/08, A61K 9/00, A61K 9/06, A61L 26/00, A61K 47/36

(54) **COMPOSITION COMPRISING STABILIZED PHYCOCYANIN AND ITS USES**
ZUSAMMENSETZUNG MIT STABILISIERTEM PHYCOCYANIN UND VERWENDUNGEN DAVON
COMPOSITION COMPRENANT DE LA PHYCOCYANINE STABILISÉE ET SES UTILISATIONS

(43) Date of publication of application: 06.01.2021
(73) Proprietor: Calicea, 75001 Paris (FR)
(72) Inventor: DELOUYA, Patrick, 75013 PARIS (FR)

(56) References cited:
- WO-A1-2014/045177
- CN-A- 106 667 794
- CN-A- 107 510 617
- KR-A- 20170 036 879

## Description

This invention is directed to a composition comprising, in a topically acceptable medium, phycocyanin and a combination of specific stabilizers thereof. It also pertains to a cosmetic or dermatological formulation comprising this composition in a cosmetic or dermatological acceptable medium, respectively. This invention is further directed to a method for reducing the signs of skin aging, comprising topically applying onto skin the above composition or cosmetic formulation, and to the above composition or dermatological formulation for its use in the treatment of wounds.

### BACKGROUND OF THE INVENTION

Phycocyanin is a water soluble protein which is one of the main pigments of cyanobacteria as for example spirulin (*Arthrospira platensis* or *Spirulina maxima*) or AFA (*Aphanizomenon Flos-Aquae*). At rest, phycocyanin has a blue colour and a red fluorescence. The pigment, respectively the chromophore, of phycocyanin is phycocyanobilin. This chromophore is a tetrapyrrol which is covalently bound to the protein by a thioether bond. Additionally to the thioether bond the chromophore interacts with the protein by hydrogen bonding which results in favorable conformation of the chromophore which is responsible for its blue color. Phycocyanin is very sensitive to temperature and pH-changes in the environment because of its polypeptide subunits. In addition, any changes to the protein-chromophore interaction leads to loss of color. Finally, the chromophore is sensitive to oxidation which may also reduce the color.

Stabilizing phycocyanin in cosmetic or dermatological formulations thus remains a challenge.

However, it would be useful to provide a stable phycocyanin formulation in view of the numerous beneficial properties of phycocyanin.

It has indeed been shown that phycocyanin is a powerful antioxidant (see for instance Jean-Yves Berthon et al., Free Radical Research, Vol. 51, No. 6, 555-567 (2017)) and could thus be useful to prevent skin damage due to the formation of reactive oxygen species (ROS) triggered by UV or pollution. ROS have indeed been known to promote the expression of matrix metalloproteinases (MMP) that induce collagen degradation in dermal skin layer. The loss of type 1 procollagen is one of the major causes of wrinkles. It has thus been suggested to use phycocyanin for alleviating the signs of skin aging.

In addition, phycocyanin is known to increase fibroblast proliferation and migration, which account for its wound healing properties (see for instance H.K. Madhyastha et al., J.Cell. Mol. Med. Vol. 12, No. 6B, 2691-2703 (2008)).

Prior attempts to stabilize phycocyanin comprise complexing phycocyanin or its chromophore with a polyphenol such as gallate esters or tannic acid, in order to protect it from external factors such as temperature and light and also against pH variations (WO 2015/090697). It has also been suggested to protect phycocyanin against photodegradation by adding ascorbic acid or a derivative, such as sodium ascorbyl phosphate, to an aqueous solution of phycocyanin (WO 2005/065697).

Nonetheless, there remains the need to provide a phycocyanin composition which is stable against degradation without detrimentally affecting the biological properties of phycocyanin.

### SUMMARY

This invention is directed to a composition comprising, in a topically acceptable medium:
(a) phycocyanin,
(b) resveratrol or a glucoside thereof in a weight ratio to phycocyanin of from 0.1:1 to 3: 1,
(c) tocopherol or its acetate in a weight ratio to phycocyanin of from 0.1:1 to 3: 1,
(d) ascorbyl methylsilanol pectinate in a weight ratio to phycocyanin of from 0.05:1 to 0.2:1,
(e) an effective amount of capryloyl glycine,
(f) pH adjusters in an effective amount to set the pH from 6.0 to 6.5, and
(g) an effective amount of a citrate phosphate buffer.

This invention also pertains to a cosmetic or dermatological formulation comprising this composition in a cosmetic or dermatological acceptable medium, respectively.

It is further directed to a cosmetic method for reducing the signs of skin aging, comprising topically applying onto skin the above composition or cosmetic formulation.

Still further, this invention pertains to the above composition or dermatological formulation for its use in the treatment of wounds.

### DETAILED DESCRIPTION

This invention pertains to a composition comprising phycocyanin in stable form.

In the context of this description, "stable" means that phycocyanin maintains a blue color, preferably blue pure or blue violet, having an maximum of absorption of light with a wavelength within an interval from 550 to 670 nm, preferably 560 to 640 nm, more preferably 580 to 620 nm, especially from 600 to 610 nm (with possibly two peaks within these ranges), when stored at a temperature of between 15 and 30°C or even between 25 and 40°C at 50-65 % RH or even at 60-75 % RH (Relative Humidity) for at least two weeks, preferably at least one month, more preferably at least two months and still preferably at least three months, in the dark (typically in an opaque container) and preferably also in day light.

Phycocyanin may be isolated from Spirulina algae according to well-known methods, for example according to the method disclosed by Seo et al. (Int. J. Mol. Sci. 2013, 14, 1778-1787), Muthulakshri\ et al. (J. Algal Biomass Utln. 2012, 3, 7-11), Hemlata et al. (J. Algal Biomass Utln. 2011, 2, (1), 30-51) or Gantar et al. (J. Biotechnol. 2012, 159 (1-2), 21-26). Alternatively, commercially available phycocyanin can be used, such as the powder marketed by DIC under the trade name Linablue^{®}. Phycocyanin may be present in an amount of 0.1 to 0.5 wt.%, preferably from 0.1 to 0.2 wt.%, in the composition of this invention.

In this composition, phycocyanin is mixed with a combination of stabilizers, namely resveratrol, ascorbyl methylsilanol pectinate, tocopherol or its acetate, capryloyl glycine and a buffer.

In the context of this description, unless otherwise specified, "resveratrol" refers both to resveratrol itself and to one of its glucosides. Resveratrol may indeed be extracted as such or in the form of resveratrol-3-O-glucoside from vegetable sources, for instance from the roots of Japanese knotweed (*Fallopia japonica*) or of *Veratrum album* or *Veratrum formosanum,* from *Yucca schidigera,* from cocoa beans or grape skin or from various fruits such as red berry, blackberry, rhubarb, dates or pomegranate. It is preferred to use *trans* resveratrol or a mixture of *cis* and *trans* resveratrol mainly comprising the *trans* isomer thereof, for instance at least 60%, preferably at least 70%, more preferably at least 80% or even at least 95% of *trans* resveratrol. Resveratrol may be obtained, for instance, from MINASOLVE under the trade name Resve^{®}. The weight ratio of resveratrol to phycocyanin ranges from 0.1:1 to 3:1 and is usually from 0.5:1 to 2.5:1. Resveratrol represents from 0.05 to 1 wt.% and preferably from 0.1 to 0.5 wt.% of the composition.

Another component of the composition of this invention is ascorbyl methylsilanol pectinate, which may be obtained from EXSYMOL under the trade name Ascorbosilane SP^{®} or Ascorbosilane C^{®}, for instance. The weight ratio of this compound to phycocyanin is usually from 0.05:1 to 0.2:1 and preferably from 0.1:1 to 0.15:1. It represents from 0.01 to 0.5 wt.% and preferably from 0.01 to 0.05 wt.% of the composition.

Still another component of the composition of this invention is tocopherol or its acetate. Tocopherol may be produced from vegetable sources such as soybean oil, preferably non-GMO oils. Mention can be made of Bioxan^{®} E1000 which is supplied by BTSA. The weight ratio of tocopherol or its acetate to phycocyanin ranges from 0.1:1 to 3:1 and is usually from 0.5:1 to 2.5:1. Tocopherol or its acetate represents from 0.05 to 1 wt.% and preferably from 0.1 to 0.5 wt.% of the composition.

A specific preservative is also included within the composition of this invention. It has indeed been found that capryloyl glycine protected efficiently this composition while avoiding any degradation of phycocyanin. Capryloyl glycine may optionally be used as an aqueous solution as that sold by MINASOLVE under the trade name CapEasy^{®}. For instance, this preservative may represent from 0.1 to 0.5 wt.% and preferably from 0.1 to 0.3 wt.% of the composition.

Finally, this composition comprises pH adjusters in an effective amount to set the pH from 6.0 to 6.5, together with a phosphate citrate buffer. The pH adjusters may comprise a mixture of an acid and a base, wherein the acid is preferably citric acid and the base is preferably sodium hydroxide. It is preferred that the pH adjusters do not comprise triethanolamine. The citrate phosphate buffer may be prepared by combining citric acid, dibasic sodium phosphate or an hydrate thereof and a pH adjuster such as sodium hydroxide. It may represent from 3 to 5 wt. % of the composition, for example.

Advantageously, the composition of this invention does not comprise sodium ascorbyl phosphate. More generally, it does not comprise any other source of vitamin C than ascorbyl methylsilanol pectinate.

The composition according to this invention is intended for topical application to skin, and it thus contains a topically acceptable medium. For the purposes of the present invention, the term "topically acceptable medium" is intended to mean a medium that is compatible with the skin. The topically acceptable medium is in particular a cosmetically or dermatologically acceptable medium, i.e. a medium that has no unpleasant odor, color or appearance, and that does not cause the user any unacceptable stinging, tautness or redness.

This medium usually comprises water, and preferably consists of water, which means that, in the latter case, the composition does not comprise any other component than phycocyanin, resveratrol, ascorbyl methylsilanol pectinate, tocopherol or its acetate, capryloyl glycine, pH adjusters, buffers and water.

The composition of this invention may be manufactured via any known method generally used in the cosmetic or dermatological fields.

This composition may be used as such or added to a cosmetic or dermatological formulation. This invention thus also pertains to a cosmetic or dermatological formulation comprising the composition as described above in a cosmetic or dermatological acceptable medium, respectively. The inventors have shown that the above composition allowed preventing phycocyanin degradation, even when this composition is combined with various other excipients and/or active agents typically found in a cosmetic or dermatological formulation.

This formulation comprises an aqueous phase and optionally a fatty phase.

The aqueous phase of this formulation comprises water and may further comprise at least one C₂-C₃₂ polyol which is in liquid form at ambient temperature. A polyol that is suitable for use in the invention may be a compound of linear, branched or cyclic, saturated or unsaturated alkyl type, bearing on the alkyl chain at least two -OH functions, in particular at least three -OH functions and more particularly at least four -OH functions. Suitable polyols are those containing from 2 to 32 carbon atoms and preferably 3 to 16 carbon atoms. Advantageously, the polyol may be chosen, for example, from ethylene glycol, pentaerythritol, trimethylolpropane, propylene glycol, 1,3-propanediol, butylene glycol, isoprene glycol, pentylene glycol, hexylene glycol, glycerol, polyglycerols such as glycerol oligomers, for instance diglycerol, and polyethylene glycols, and mixtures thereof. According to a preferred embodiment, said polyol is chosen from ethylene glycol, pentaerythritol, trimethylolpropane, propylene glycol, glycerol, polyglycerols, polyethylene glycols and mixtures thereof. Most preferably, the polyol comprises glycerol and/or propylene glycol.

In addition, the aqueous phase may comprise one or more gelling agents, such as cellulose and its derivatives; modified starches; carraghenan; agar agar; xanthan gum; plant gums such as guar or locust bean gum; synthetic polymers such as optionally crosslinked homopolymers of sodium acrylate, copolymers of (meth)acrylic acid or (hydroxy)alkyl esters thereof with at least one other monomer such as 2-acrylamido-2-methylpropane sulfonic acid, wherein these copolymers are optionally crosslinked; and mixtures thereof. In the above list, the chemical groups within brackets may or not be present.

In case it is present, the fatty phase of the cosmetic or dermatological formulation includes any liquid fatty substance, generally oils, and/or solid fatty substance like waxes or pasty compounds.

The term "oil" is intended to mean any fatty substance that is in liquid form at ambient temperature and atmospheric pressure. The oils may be volatile or nonvolatile. These oils may be of animal, plant, mineral or synthetic origin. They can be selected from hydrocarbon oils, silicon oils, ester oils (including triglycerides) and their mixtures.

Among the volatile hydrocarbon-based oils containing from 8 to 16 carbon atoms, mention may be made in particular of branched C₈-C₁₆ alkanes, for instance C₈-C₁₆ isoalkanes (also known as isoparaffins), such as isododecane, isodecane and isohexadecane. Mention may also be made of volatile linear alkanes comprising from 10 to 13 carbon atoms, and mixtures thereof. Volatile linear silicone oils that may be mentioned include linear volatile silicone oils such as hexamethyldisiloxane, octamethyltrisiloxane, decamethyltetrasiloxane, tetradecamethyl-hexasiloxane, hexadecamethylheptasiloxane and dodecamethylpentasiloxane. Volatile cyclic silicone oils that may be mentioned include hexamethylcyclotrisiloxane, octamethylcyclotetrasiloxane, decamethylcyclopentasiloxane and dodecamethylcyclohexasiloxane.

The nonvolatile oils may, in particular, be chosen from: hydrocarbon-based oils of plant origin, such as liquid triglycerides of fatty acids containing from 4 to 10 carbon atoms, for instance heptanoic or octanoic acid triglycerides, or alternatively, for example, sunflower oil, maize oil, soybean oil, marrow oil, grapeseed oil, sesame seed oil, hazelnut oil, apricot oil, macadamia oil, castor oil, avocado oil, caprylic/capric acid triglycerides, jojoba oil and shea butter oil; synthetic ethers containing from 10 to 40 carbon atoms, such as dicaprylyl ether; synthetic esters, such as the oils having formula R₁COOR₂, in which Ri represents a linear or branched fatty acid residue comprising from 1 to 40 carbon atoms and R₂ represents a hydrocarbon-based chain, which is in particular branched, containing from 1 to 40 carbon atoms, provided that R₁+R₂ is ≧10; fatty alcohols that are liquid at ambient temperature, with a branched and/or unsaturated carbon-based chain containing from 12 to 26 carbon atoms; C₁₂-C₂₂ higher fatty acids; non-phenyl silicone oils, for instance caprylyl methicone; phenyl silicone oils, for instance phenyl trimethicones, phenyl dimethicones, phenyltrimethylsiloxydiphenylsiloxanes, diphenyl dimethicones, diphenylmethyldiphenyl-trisiloxanes and 2-phenylethyl trimethylsiloxysilicates, dimethicones or phenyl trimethicone with a viscosity of less than or equal to 100 cSt, and trimethyl-pentaphenyl-trisiloxane, and mixtures thereof; and also mixtures of these various oils.

The liquid fatty phase may contain other compounds, such as oil gelling agents and/or structuring agents. These compounds may be chosen in particular from gums, such as silicone gums (dimethiconol); silicone resins; and silicone elastomers; and mixtures thereof.

The kind and amount of fatty substances mentioned above can be chosen by the person skilled in the art so as to prepare a formulation having the desired properties, for example of consistency or texture.

The formulation may also comprise one or more additional agents chosen from antioxidants, preservatives, fragrances, dyes, pigments, surfactants, chelators, fillers and any other ingredient normally used in the cosmetic and/or dermatological field, and mixtures thereof.

Fillers may be mineral or organic and of any shape, i.e. platelet-shaped, spherical or oblong, irrespective of the crystallographic form. Mention may be made of silica, talc, mica, kaolin, lauroyl lysine, starch, boron nitride, PTFE powders, PMMA powders, methylsilsesquioxane resin powders, hollow silicone resin hemispherical particles, barium sulfate, precipitated calcium carbonate, magnesium carbonate, magnesium hydrogen carbonate, hydroxyapatite, glass or ceramic microcapsules, and metal soaps derived from organic carboxylic acids containing from 8 to 22 carbon atoms and preferably from 12 to 18 carbon atoms, for example zinc stearate, magnesium stearate, lithium stearate, zinc laurate or magnesium myristate.

Depending on its intended use, this formulation may further include at least one active agent chosen from moisturizers, wound healing agents, anti-aging agents, UV screening agents and their mixtures.

Examples of active agents are the following:
- a metalloproteinase inhibitor, and/or
- a humectant, and/or
- a myorelaxant, and/or
- an agent that stimulates the synthesis of collagen, and/or
- an agent that stimulates the synthesis of elastin, and/or
- an agent that stimulates the synthesis of glycosaminoglycans,
- an agent that stimulates the synthesis of fibronectin, and/or
- an agent that stimulates fibroblast proliferation, and/or
- an agent that stimulates keratinocyte proliferation and/or differentiation, and/or
- a desquamating agent, and/or
- an agent that improves skin barrier function, and/or
- a depigmenting agent, and/or
- an agent that promotes the maturation of the horny envelope, and/or
- an agent that promotes the microcirculation of the skin or scalp, and/or
- a calmative or anti-irritant.

The above formulation may be prepared according to the techniques that are well known to those skilled in the art. In addition, it may be in any presentation form conventionally used for the intended applications and in particular in the form of a dispersion of aqueous gel or lotion type, of an emulsion of liquid to semi-solid consistency, obtained by dispersing a fatty phase in an aqueous phase (O/W) or conversely (W/O), or alternatively of a multiple emulsion (W/O/W or O/W/O). These formulations are prepared according to usual methods. Aqueous gels are preferred for use in this invention.

The emulsions may comprise at least one emulsifier chosen from amphoteric, anionic, cationic and nonionic emulsifiers, used alone or as a mixture, which are appropriately chosen according to the emulsion to be obtained (W/O or O/W). Examples of emulsifiers that may be mentioned for the O/W emulsions include nonionic surfactants, and especially esters of polyols and of fatty acids with a saturated or unsaturated chain containing, for example, from 8 to 24 carbon atoms and better still from 12 to 22 carbon atoms, and the oxyalkylenated derivatives thereof, i.e. derivatives containing oxyethylenated and/or oxypropylenated units, such as the glyceryl esters of C8-C24 fatty acids, and the oxyalkylenated derivatives thereof; polyethylene glycol esters of C8-C24 fatty acids, and the oxyalkylenated derivatives thereof; sorbitol esters of C8-C24 fatty acids, and the oxyalkylenated derivatives thereof; sugar (sucrose, glucose or alkylglucose) esters of C8-C24 fatty acids, and the oxyalkylenated derivatives thereof; fatty alcohol ethers; sugar ethers of C8-C24 fatty alcohols, and mixtures thereof.

Needless to say, a person skilled in the art will take care to select this or these optional additional compound(s), and/or the amount thereof, such that the biological properties of the formulation according to the invention are not, or are not substantially, adversely affected by the envisaged addition, and such that the formulation is suitable for a topical application.

This formulation may be used as a skin care, sunscreen or make-up product. More specifically, the skin care product may be an anti-aging, a moisturizing or a cleansing product for the face, for the hands, for the feet or for the body (for example day creams, night creams, protective or care body milks, after-sun milks, skincare lotion, gel or foam, aftershave products or artificial tanning compositions). The make-up product may be a foundation or a concealer, for instance.

This invention is more particularly directed to a cosmetic method for reducing the signs of skin aging, comprising topically applying onto skin the composition as described above or a cosmetic formulation comprising the same. It is further directed to a composition as described above or a dermatological formulation comprising the same, for its use in the treatment of burns or wounds.

The topical administration consists of the external application to the skin of the cosmetic and/or dermatological composition or formulation according to customary techniques. The method may comprise a single application. According to another embodiment, the application is repeated, for example 2 to 3 times per day for one day or more, and generally for an extended period of at least 4 weeks, or even 4 to 15 weeks, with, where appropriate, one or more periods of stoppage.

Throughout the description, including the claims, the expression "comprising a" should be understood as being synonymous with "comprising at least one", unless otherwise specified. The expressions "between ... and ..." and "ranging from ... to ..." should be understood as meaning limits included, unless otherwise specified.

The examples which follow are presented by way of illustration and without implied limitation of the invention. The compounds are, as the case may be, mentioned by their chemical names or their INCI names.

### EXAMPLES

### Example 1 : Preparation of a composition according to the invention

A composition was prepared by mixing the following constituents in the amounts indicated below:

| Constituent | Amount (%) |
|---|---|
| WATER | 68.6 |
| GLYCERIN | 29.4 |
| Phycocyanin | 0.2 |
| RESVERATROL | 0.5 |
| TOCOPHEROL ACETATE | 0.5 |
| ASCORBYL METHYLSILANOL PECTINATE (1%) & METHYLPROPANEDIOL & WATER | 3.0 |
| Citrate phosphate buffer | 5.0 |
| CAPRYLOYL GLYCINE (30%) & WATER & SODIUM BICARBONATE | 1.0 |
| CITRIC ACID | qsp pH 6.0-6.5 |
| SODIUM HYDROXIDE | |
| Total | 100 |

### Example 2: Stability tests

### 2A- Stability of phycocyanin in the composition of this invention

The composition of Example 1 (Comp 1) was stored under the following Relative Humidity (RH) and temperature (T) conditions:
C1: T=25°C, RH=65%
C2: T=30°C, RH=60%
C3: T=40°C, RH=75%
for three months.

Two additional experiments were conducted on the same composition stored under ambient conditions (RH = 54.4%; T = 18.9°C), both in a dark bottle (CD) and in daylight (CL).

In all cases, analyses were performed at the time of preparing the composition (To), then after two weeks (T_{2w}).

Composition Comp 1 appeared as a blue liquid under all conditions. In addition, its pH (which was originally of 6.06) did not vary by more than 3% after two weeks of storage under any condition tested.

### 2B- Stability of phycocyanin in the presence of various excipients

The same experiment as above was conducted on formulation F1 comprising, in addition to the above composition Comp 1, xanthan gum (0.5%), squalane (1%), Poloxamer 407 (1.0%) and propylene glycol (5.0%). The amount of glycerin was lowered to 2% and the amount of water was adjusted consequently. A comparative formulation F1A was also prepared, which was identical to F1 except that it was free of resveratrol, tocopherol acetate, ascorbyl methylsilanol pectinate, citrate phosphate buffer and capryloyl glycine.

The results of this experiment are summarized below:

| | Time | Color | | pH | |
|---|---|---|---|---|---|
| | | F1 | F1A | F1 | F1A |
| C1 | To | Blue | Blue | 6.23 | 6.41 |
| | T_{2W} | Blue | Turquoise | 6.48 | 4.93 |
| C2 | To | Blue | Blue | 6.23 | 6.41 |
| | T_{2w} | Grey blue | Blue-green | 6.17 | 5.35 |
| C3 | To | Blue | Blue | 6.23 | 6.41 |
| | T_{2w} | Grey blue | Light green | 6.27 | 5.14 |
| CD | To | Blue | Blue | 6.23 | 6.41 |
| | T_{2w} | Blue | Blue-green | 6.61 | 5.18 |
| CL | To | Blue | Blue | 6.23 | 6.41 |
| | T_{2w} | Blue | Blue | 6.58 | 5.58 |

As can be seen from this Table, the comparative formulation (F1A) turns to green when stored at 30°C or higher and its pH varies by up to 23%, whereas the formulation of this invention (Fl) remains blue and its pH does not vary by more than 6%. This experiment demonstrates that the stabilizing system used in this invention effectively protects phycocyanin from thermal and light-induced degradation in the presence of usual excipients.

### 2C- Stability of phycocyanin in the presence of other active agents

The same experiment as above was conducted on two formulations (F2 and F2A) comprising, 4% of skin care active agents in addition to the above formulations (F1 and F1A), respectively.

The results of this experiment are summarized below:

| | Time | Color | | pH | |
|---|---|---|---|---|---|
| | | F2 | F2A | F2 | F2A |
| C1 | To | Blue | Blue | 6.12 | 6.23 |
| | T_{2w} | Blue | Turquoise | 6.79 | 5.58 |
| C2 | To | Blue | Blue | 6.12 | 6.23 |
| | T_{2w} | Blue | Turquoise | 6.64 | 5.50 |
| C3 | To | Blue | Blue | 6.12 | 6.23 |
| | T_{2w} | Blue-green | Green | 6.44 | 5.54 |
| CD | To | Blue | Blue | 6.12 | 6.23 |
| | T_{2w} | Blue | Green | 6.17 | 5.58 |
| CL | To | Blue | Blue | 6.12 | 6.23 |
| | T_{2w} | Blue | Blue-green | 6.54 | 6.51 |

As can be seen from this Table, except at 40°C where it slightly turns to green, the formulation of this invention (F2) remains stable under all conditions. Conversely, the comparative formulation (F2A) does not remain blue under any condition.

### Example 3 : Cosmetic formulation

A formulation comprising the following ingredients can be prepared according to usual methods.

| | | |
|---|---|---|
| Stabilized phycocyanin | | 4.00 % |
| Emulsifiers | | 6.00 % |
| Oils and fats | | 10.00 % |
| Glycerin | | 6.00 % |
| Thickeners | | 2.50 % |
| Perfume | | qs |
| Water | qsp | 100,00 % |

This formulation may be applied to skin in the morning and/or in the evening to fight against signs of skin ageing.

### Example 4: Wound healing formulation

A formulation P1 comprising the following ingredients was prepared according to usual methods: water, stabilized phycocyanin, polyhydric alcohols, squalane, surfactants, wanthan gum, soothing agents, preservatives, perfume.

The wound healing effect of this formulation was tested on human skin explants *ex vivo.* For this purpose, 6 rectangular explants of 10x15 mm were prepared from abdominal plastic surgery of a Caucasian woman aged 35. The explants were split into 3 batches, among which:
- batch B corresponded to the wounded control, i.e. an explant onto which two wounds were made by means of a punch of 3.5mm diameter at J0, and
- batch BP1 corresponded to a wounded explant onto which 3µL of formulation P1 were applied (2mg/cm²) at J0.

The explants of all batches were put into a BEM medium at 37°C in a moist atmosphere enriched in 5% CO2. Half of the culture medium was renewed at J2, J3, J6 and J8. An additional 3 µL amount of formulation P1 was further applied at the same times.

After 10 days, 3 explants from each batch were picked-up from the medium and cut into two pieces, one of which was treated with a buffered formol solution for 24 hours, then dehydrated and impregnated with paraffin and formed as a block. The blocks were then cut to 5 µm slices with a microtome, stained with a Masson-Goldner trichrome kit and mounted on Superfrost^{®} glass slides.

All the samples were analyzed with an optical microscope (Leica DMLB or Olympus BX43). Pictures were taken with an Olympus DP72 camera and the Cell D software.

### Results:

The width of the epidermal wound was measured on two different areas of each explant: the mean width measured on samples from batch BP1 was 39% smaller than that measured on the samples from batch B (p<0.05). This contraction of the wound accounts for the activation of contractile properties of the myofibroblasts, which enables wound coating by neo-epidermis.

## Claims

1. Composition comprising, in a topically acceptable medium:
(a) phycocyanin,
(b) resveratrol or a glucoside thereof in a weight ratio to phycocyanin of from 0.1:1 to 3:1,
(c) tocopherol or its acetate in a weight ratio to phycocyanin of from 0.1:1 to 3:1,
(d) ascorbyl methylsilanol pectinate in a weight ratio to phycocyanin of from 0.05:1 to 0.2:1,
(e) an effective amount of capryloyl glycine,
(f) pH adjusters in an effective amount to set the pH from 6.0 to 6.5, and
(g) an effective amount of a citrate phosphate buffer.

2. Composition according to claim 1, **characterized in that** it comprises:
(a) from 0.1 to 0.5 wt.% of phycocyanin,
(b) from 0.05 to 1 wt.% of resveratrol or a glucoside thereof,
(c) from 0.05 to 1 wt.% of tocopherol or its acetate,
(d) from 0.01 to 0.5 wt.% of ascorbyl methylsilanol pectinate,
(e) an effective amount of capryloyl glycine,
(f) pH adjusters in an effective amount to set the pH from 6.0 to 6.5, and
(g) an effective amount of a citrate phosphate buffer.

3. Composition according to claim 1 or 2, **characterized in that** resveratrol is *trans* resveratrol or a mixture of *cis* and *trans* resveratrol mainly comprising the *trans* isomer thereof, for instance at least 60%, preferably at least 70%, more preferably at least 80% or even at least 95% of *trans* resveratrol.

4. Composition according to any one of claims 1 to 3, **characterized in that** the pH adjusters comprise a mixture of an acid and a base, wherein the acid is preferably citric acid and the base is preferably sodium hydroxide.

5. Composition according to any one of claims 1 to 4, **characterized in that** the citrate phosphate buffer represents from 3 to 5 wt. % of the composition.

6. Composition according to any one of claims 1 to 5, **characterized in that** the topically acceptable medium comprises, and preferably consists of, water.

7. A cosmetic or dermatological formulation comprising the composition according to any one of claims 1 to 6 in a cosmetic or dermatological acceptable medium, respectively.

8. A cosmetic method for reducing the signs of skin aging, comprising topically applying onto skin the composition according to any one of claims 1 to 6 or a cosmetic formulation according to claim 7.

9. Composition according to any one of claims 1 to 6 or a dermatological formulation according to claim 7, for its use in the treatment of wounds.

## Patentansprüche

1. Zusammensetzung, umfassend in einem topisch verträglichen Medium:
(a) Phycocyanin,
(b) Resveratrol oder ein Glucosid davon in einem Gewichtsverhältnis zu Phycocyanin von 0,1:1 bis 3:1,
(c) Tocopherol oder sein Acetat in einem Gewichtsverhältnis zu Phycocyanin von 0,1:1 bis 3:1,
(d) Ascorbylmethylsilanolpektinat in einem Gewichtsverhältnis zu Phycocyanin von 0,05:1 bis 0,2:1,
(e) eine wirksame Menge Capryloylglycin,
(f) pH-Einstellmittel in einer wirksamen Menge, um den pH-Wert auf 6,0 bis 6,5 einzustellen, und
(g) eine wirksame Menge eines Citrat-Phosphat-Puffers.

2. Zusammensetzung nach Anspruch 1, **dadurch gekennzeichnet, dass** sie umfasst:
(a) 0,1 bis 0,5 Gew.-% Phycocyanin,
(b) 0,05 bis 1 Gew.-% Resveratrol oder ein Glucosid davon,
(c) 0,05 bis 1 Gew.-% Tocopherol oder sein Acetat,
(d) 0,01 bis 0,5 Gew.-% Ascorbylmethylsilanolpektinat,
(e) ine wirksame Menge Capryloylg lycin,
(f) pH-Einstellmittel in einer wirksamen Menge, um den pH-Wert auf 6,0 bis 6,5 einzustellen, und
(g) eine wirksame Menge eines Citrat-Phosphat-Puffers.

3. Zusammensetzung nach Anspruch 1 oder 2, **dadurch gekennzeichnet, dass** Resveratrol trans-Resveratrol oder eine Mischung aus cis- und trans-Resveratrol ist, die hauptsächlich das trans-Isomer davon umfasst, beispielsweise mindestens 60 %, vorzugsweise mindestens 70 %, stärker bevorzugt mindestens 80 % % oder sogar mindestens 95 % Trans-Resveratrol.

4. Zusammensetzung nach einem der Ansprüche 1 bis 3, **dadurch gekennzeichnet, dass** die pH-Einstellmittel ein Gemisch aus einer Säure und einer Base umfassen, wobei die Säure vorzugsweise Citronensäure und die Base vorzugsweise Natriumhydroxid ist.

5. Zusammensetzung nach einem der Ansprüche 1 bis 4, **dadurch gekennzeichnet, dass** der Citrat-Phosphat-Puffer 3 bis 5 Gew.-% der Zusammensetzung darstellt.

6. Zusammensetzung nach einem der Ansprüche 1 bis 5, **dadurch gekennzeichnet, dass** das topisch verträgliche Medium Wasser umfasst und vorzugsweise daraus besteht.

7. Kosmetische oder dermatologische Formulierung, enthaltend die Zusammensetzung nach einem der Ansprüche 1 bis 6 in einem kosmetisch bzw. dermatologisch verträglichen Medium.

8. Kosmetisches Verfahren zur Verringerung der Zeichen der Hautalterung, umfassend das topische Auftragen der Zusammensetzung nach einem der Ansprüche 1 bis 6 oder einer kosmetischen Formulierung nach Anspruch 7 auf die Haut.

9. Zusammensetzung nach einem der Ansprüche 1 bis 6 oder eine dermatologische Formulierung nach Anspruch 7, zu ihrer Verwendung bei der Behandlung von Wunden.

## Revendications

1. Composition comprenant, dans un milieu acceptable par voie topique :
(a) une phycocyanine,
(b) du resvératrol ou un glucoside de celui-ci en un rapport en poids à la phycocyanine de 0,1/1 à 3/1,
(c) du tocophérol ou son acétate en un rapport en poids à la phycocyanine de 0,1/1 à 3/1,
(d) du pectinate d'ascorbyl-méthylsilanol en un rapport en poids à la phycocyanine de 0,05/1 à 0,2/1,
(e) une quantité efficace de capryloyl-glycine,
(f) des agents d'ajustement du pH en une quantité efficace pour établir le pH à une valeur de 6,0 à 6,5, et
(g) une quantité efficace d'un tampon citrate-phosphate.

2. Composition selon la revendication 1, **caractérisée en ce qu'**elle comprend :
(a) 0,1 à ,5 % en poids de phycocyanine,
(b) 0,05 à 1 % en poids de resvératrol ou d'un glucoside de celui-ci,
(c) 0,05 à 1 % en poids de tocophérol ou de son acétate,
(d) 0,01 à 0,5 % en poids de pectinate d'ascorbyl-méthylsilanol,
(e) une quantité efficace de capryloyl-glycine,
(f) des agents d'ajustement du pH en une quantité efficace pour établir le pH à une valeur de 6,0 à 6,5, et
(g) une quantité efficace d'un tampon citrate-phosphate.

3. Composition selon la revendication 1 ou 2, **caractérisée en ce que** le resvératrol est du resvératrol *trans* ou un mélange de resvératrol *cis* et *trans* comprenant principalement l'isomère *trans* de celui-ci, par exemple au moins 60 %, de préférence au moins 70 %, mieux encore au moins 80 % ou même au moins 95 % de resvératrol *trans.*

4. Composition selon l'une quelconque des revendications 1 à 3, **caractérisée en ce que** les agents d'ajustement du pH comprennent un mélange d'un acide et d'une base, dans laquelle l'acide est de préférence l'acide citrique et la base est de préférence l'hydroxyde de sodium.

5. Composition selon l'une quelconque des revendications 1 à 4, **caractérisée en ce que** le tampon citrate-phosphate représente 3 à 5 % en poids de la composition.

6. Composition selon l'une quelconque des revendications 1 à 5, **caractérisée en ce que** le milieu acceptable par voie topique comprend, et de préférence consiste en, de l'eau.

7. Formulation cosmétique ou dermatologique comprenant la composition selon l'une quelconque des revendications 1 à 6 dans un milieu acceptable du point de vue cosmétique ou dermatologique respectivement.

8. Méthode cosmétique pour réduire les signes du vieillissement de la peau, comprenant l'application topique sur la peau de la composition selon l'une quelconque des revendications 1 à 6 ou d'une formulation cosmétique selon la revendication 7.

9. Composition selon l'une quelconque des revendications 1 à 6 ou formulation dermatologique selon la revendication 7, pour son utilisation dans le traitement de plaies.
